# EUROPEAN PATENT SPECIFICATION

(11) **EP 2 752 659 B1**
(45) Date of publication and mention of the grant of the patent: **18.10.2017**
(21) Application number: 12827009.7
(22) Date of filing: 29.08.2012
(51) Int. Cl.: G01N 27/62, G01N 27/64

(54) **MALDI MASS ANALYSIS METHOD**
MALDI-MASSENANALYSEVERFAHREN
PROCÉDÉ D'ANALYSE PAR SPECTROMÉTRIE DE MASSE DE TYPE MALDI

(30) Priority: 31.08.2011 JP 2011188615
(43) Date of publication of application: 09.07.2014
(73) Proprietor: The Noguchi Institute, Tokyo 1730003 (JP)
(72) Inventor: AMANO Junko, Tokyo 173-0003 (JP); OKUMURA Hisako, Tokyo 173-0003 (JP)
(74) Representative: dompatent von Kreisler Selting Werner - Partnerschaft von Patent- und Rechtsanwälten mbB
(86) International application number: PCT/JP2012/071772
(87) International publication number: WO 2013/031797

(56) References cited:
- WO-A1-2011/081180
- JP-A- 2008 051 790
- JP-A- 2009 257 844
- JP-A- 2010 203 925
- JP-A- 2011 053 086
- TAKASHI NISHIKAZE ET AL: "Negative-ion MALDI-MSfor discrimination of 2,3- and 2,6-sialylation on glycopeptides labeled with a pyrene derivative", JOURNAL OF CHROMATOGRAPHY B: BIOMEDICAL SCIENCES & APPLICATIONS, ELSEVIER, AMSTERDAM, NL, vol. 879, no. 17, 27 October 2010 (2010-10-27), pages 1419-1428, XP028203532, ISSN: 1570-0232, DOI: 10.1016/J.JCHROMB.2010.10.032 [retrieved on 2010-11-04]
- ULLMER ROMAN ET AL: "Derivatization by 6-aminoquinolyl-N-hydroxysuccinimidyl carbamate for enhancing the ionization yield of small peptides and glycopeptides in matrix-assisted laser desorption/ionization and electrospray ionization massspectrometry", RAPID COMMUNICATIONS IN MASS SPECTROMETRY, JOHN WILEY & SONS, GB, vol. 20, no. 9, 1 January 2006 (2006-01-01), pages 1469-1479, XP009098545, ISSN: 0951-4198, DOI: 10.1002/RCM.2464
- SUZUKI H ET AL: "Analysis of 1-aminopyrene-3,6,8-trisulfonate-derivatiz ed oligosaccharides by capillary electrophoresis with matrix-assisted laser desorption/ionization time-of-flight mass spectrometry", ANALYTICAL CHEMISTRY, AMERICAN CHEMICAL SOCIETY, vol. 69, no. 22, 15 November 1997 (1997-11-15), pages 4554-4559, XP002512792, ISSN: 0003-2700, DOI: 10.1021/AC970090Z
- AMANO JUNKO ET AL.: 'Derivatization with 1-Pyrenyldiazomethane Enhances Ionization of Glycopeptides but Not Peptides in Matrix-Assisted Laser Desorption/Ionization Mass Spectrometry' ANALYTICAL CHEMISTRY vol. 82, no. 20, 15 October 2010, pages 8738 - 8743, XP055143878

## Description

### TECHNICAL FIELD

The present invention relates to a mass spectrometry method, more specifically to a MALDI mass spectrometry method that is characterized in obtaining a quantitative mass spectrum for the multiple different molecules to be measured from an arbitrary point where a signal is detected without measuring and integrated averaging the entire sample to be measured. The multiple different molecules to be measured are selected from the group consisting of a saccharide mixture, a glycopeptide mixture, a glycopeptide-peptide mixture, a glycoprotein mixture, and a glycoprotein-protein mixture.

### BACKGROUND ART

A mass spectrometry (this may be abbreviated as MS) method is a method of ionizing a sample including a molecule to be measured, separating and detecting an ion derived from the molecule to be measured by mass-to-charge ratio (mass/charge (m/z)), and observing information concerning a chemical structure of the molecule to be measured.

In MS, ionization of a sample is an important process of determining whether an analysis can be executed, influencing quality of a spectrum, and many ionization methods for effectively ionizing a sample have been developed. Recently, in ionization of a biopolymer, matrix-assisted laser desorption/ionization (this may be abbreviated as MALDI) method which is a soft ionization method, and electrospray ionization (this may be abbreviated as ESI) have been mainly used. A mass spectrometer using these ionization methods can measure a smaller amount of sample than NMR, etc. and is widely used in a biotechnology field.

In MALDI-MS, for example, analysis of a saccharide, etc. is especially difficult compared with a peptide and a protein, etc. One of the root causes is efficiency of ionization of a saccharide is extremely poor compared with a peptide, etc. and high sensitive analysis is difficult.

Since it has been reported that ionization efficiency of a sample in MALDI-MS is different due to factors such as a kind of matrix to be used, an additive to a matrix, solvent, a specific matrix for measuring with high sensitivity has been developed (Patent Documents 1 to 3, Non-Patent Documents 1 to 2).

Alternatively, since ionization efficiency is influenced by characteristics of a molecule to be measured itself, it has been known that ionization efficiency is improved by derivatization of the sample to be measured, methylation of a hydroxyl group of a glycan, and derivatization of a reduced terminal or sialic acid of a glycan (for example, Patent Documents 4 to 5).

Analyzing a molecule to be measured by converting to another molecule after previously derivatization of the molecule to be measured can obtain correct information concerning the molecule to be measured, with sensitivity of the molecule to be measured increased. Further, in the case that, in a sample to be measured, multiple molecules to be measured are present or a molecule other than the molecule to be measured coexists, a measuring method to obtain more specific or correct information has been thought, by utilizing that a specific molecule in the sample to be measured is only derivatized to distinguish with other molecule.

Especially, relating to analysis measuring a property in a part of a molecule to be measured, measuring a fragment of the molecule to be measured, not inhibiting information concerning the molecule to be measured with information concerning a derivatizing agent to be used for derivatization, and able to remove only information concerning a derivatizing agent, derivatization of the molecule to be measured is preferably used.

For example, in Non-Patent Document 3, a method utilizing matrix-assisted laser desorption/ionization mass spectrometry (MALDI-MS) in which a sample is mounted on a sample-supporting member after previously derivatization of a glycan by using 4-aminobenzoic acid ethyl ester, 2-aminopyridine, 1-pyrenebutanoic acid hydrazide (PBH), etc. has been suggested.

However, since an ionization mechanism of MALDI has not been completely solved, it is frequent that selecting a derivatizing agent or matrix to be measured, or a preparing method of a sample to be measured is based on experience knowledge. Generally, in a solid matrix to be mainly used in MALDI, it has been thought that a matrix and sample are necessary to be mixed well and thus a mixed crystal forms. But since the crystal is heterogeneous since it is a solid crystal, and an ion derived from the molecule to be measured cannot be obtained from all places where a crystal is formed, an ion derived from the molecule to be measured can be only obtained when a laser is irradiated on only part of a formed crystal.

Thus, normally, a representing spectrum is obtained by repeating laser irradiation on multiple times (several tens to several hundreds), and integrated averaging the spectra obtained individually. In a quantitative measurement, it is frequent that integration of 1000 to 3000 laser shots is performed. At that time, since there is a possibility that a measurement by fixing one point on a surface of a sample to be measured obtains a not-representing spectrum influenced by unevenness of the surface, a method for integrating while moving a laser irradiation position is used.

At that time, an automatic measurement can integrate multiple measurement points at constant intervals (Non-Patent Document 4). However, a place where an ion is formed is extremely limited according to a condition of a sample to be measured, the automatic measurement can obtain the spectra which is poor and not quantitative, as a result.

Alternatively, a method obtaining a mass spectrum with high quality for searching sweet spots in which an ion derived from a molecule to be measured is obtained with high sensitivity has been present. But, where sweet spots are present in the crystal is different according to the molecule to be measured, each sweet spot can be only searched by experience.

Further, since the positions of sweet spots are different according to molecules to be measured even if the sweet spot is searched and laser irradiation is performed thereon, a quantitative measurement of multiple molecules to be measured was difficult in the sample to be measured prepared by conventional preparing methods. For example, it was difficult to obtain each contained ratio of the multiple molecules to be measured in the sample accurately.

A generally established method to separate a mixture of a peptide and glycopeptide as a single molecule to be measured has not existed. Especially, separation and purification is extremely difficult when an amount of the sample is extremely minute.

Thus, further, in glycan structure analysis of a glycoprotein or glycopeptide, it is general that, in a mass spectrometry method, a glycan is temporarily separated from a peptide and a free glycan is purified. This method has problems that a large amount of the sample is needed due to low sensitivity, operation time requires much time, and the sample is consumed, etc.

Thus, with respect to a sample containing a molecule to be measured whose amount is extremely minute, a mass spectrometry method to measure a molecule to be measured quantitatively within a short period of time with high sensitivity and good efficiency has been especially desired, but methods which satisfy above condition sufficiently have been not present.

### PRIOR ART REFERENCES

### Patent Documents

Patent Document 1: Japanese Patent Laid-Open Publication No. 2008-261824
Patent Document 2: Japanese Patent Laid-Open Publication No. 2008-261825
Patent Document 3: Japanese Patent Laid-Open Publication No. 2001-013110
Patent Document 4: Japanese Patent Laid-Open Publication No. 2008-051790
Patent Document 5: WO 2006/109485

### Non Patent Documents

Non-Patent Document 1: Snovida, S.I. et al., J. Am. Soc. Mass spectrom. 19 (2008) 1138-1146
Non-Patent Document 2: Fukuyama, Y. et al., Anal. Chem. 80 (2008) 2172-2179
Non-Patent Document 3: Sugahara, D. et al., Anal. Sci. 19 (2003) 167-169
Non-Patent Document 4: Takahashi et al., Bunseki 7 (2007) 328-335

### SUMMARY OF THE INVENTION

### PROBLEMS TO BE SOLVED BY THE INVENTION

The present invention has been accomplished in view of the above-mentioned background art, and an object thereof is to provide a mass spectrometry method that can be used for a general MALDI mass spectrometry method, and can measure multiple different molecules to be measured that are contained in a sample quantitatively within a short period of time with good efficiency, compared with conventional methods. The multiple different molecules are selected from the group consisting of a saccharide mixture, a glycopeptide mixture, a glycopeptide-peptide mixture, a glycoprotein mixture, and a glycoprotein-protein mixture.

Further, this is more specifically to provide a mass spectrometry method which can realize a quantitative measurement within a short period of time by irradiating a laser to an arbitrary point which detects a signal of a part of a sample to be measured without measuring and integrated averaging the entire sample to be measured.

According to the above, this is to provide a mass spectrometry method which can obtain information concerning the chemical structures with high reliability even if an amount of the sample containing the molecules to be measured is extremely minute.

In addition, this is to provide a mass spectrometry method which can suitably obtain information useful for elucidation of the function or elucidation of pathological conditions by applying to the "molecules derived from a living body or molecules in a living body sample" with a minute amount such as a saccharide, a glycoprotein, a glycopeptide, etc.

### MEANS TO SOLVE THE PROBLEMS

The present inventors have earnestly investigated to solve the above-mentioned problems, and as a result, they have found that among multiple molecules to be measured contained in a sample to be measured, even if glycopeptides having different glycan structures or a peptide(s) having no saccharide is/are present therein, if the structures at the peptide portions are the same with each other, a point which detects a signal or a position which becomes a sweet spot excellent in ionization efficiency is common in the MALDI matrix crystal prepared onto a sample-supporting member.

They have also found that among the multiple molecules to be measured, even if glycopeptides having different structures at the peptide portions or a saccharide(s) having no peptide is/are present therein, if a derivatizing reaction is applied to the sample using the common derivatizing agent to carry out the common derivatization to the multiple molecules to be measured, not only ionization efficiency of the molecules to be measured is extremely improved, but also "a point which detects a signal including a sweet spot" of the multiple molecules to be measured is formed at the common position of the sample to be measured.

They have further found that quantitation of the multiple molecules to be measured can be carried out within a shorter period of time with good efficiency by irradiating a laser onto an arbitrary point which detects a signal, which is a part of the sample, even when the measurement is not carried out by irradiating a laser to the entire surface of the prepared sample to be measured with multiple points for a long period of time, whereby they have accomplished the present invention.

That is, the present invention is directed to
[1] A MALDI mass spectrometry method for a sample containing multiple different molecules to be measured, which is a MALDI mass spectrometry method characterized in that the multiple different molecules to be measured are a saccharide mixture, a glycopeptide mixture, a glycopeptide-peptide mixture, a glycoprotein mixture or a glycoprotein-protein mixture, and that a quantitative mass spectrum of the multiple different molecules to be measured is obtained from an arbitrary point in the sample to be measured where a signal is detected without measuring and integrated averaging the entire sample to be measured. The multiple different molecules to be measured either are reacted with a common derivatizing agent, said derivatizing agent being a condensed polycyclic compound having a reactive functional group capable of bonding the condensed polycyclic portion and the molecules to be measured, or have a common peptide or protein portion
[2] The MALDI mass spectrometry method described in [1], wherein the multiple molecules to be measured are a glycopeptide mixture, peptide portions of the glycopeptide are the same with each other, or are a glycopeptide-peptide mixture, peptide portions of the glycopeptide are the same with each other, and the peptide portion of the glycopeptide and the peptide are the same with each other.
[3] The MALDI mass spectrometry method described in [1], wherein the multiple molecules to be measured are a saccharide mixture, and a sample to be measured is prepared by reacting the sample with a condensed polycyclic compound, and crystallizing the obtained molecules to be measured labeled with the condensed polycyclic compound and a matrix.
[4] The MALDI mass spectrometry method described in [1], wherein the multiple molecules to be measured are a glycopeptide-peptide mixture, the peptide portions of the glycopeptide are the same with each other, the peptide portion of the glycopeptide and the peptide are the same with each other, and a sample to be measured is prepared by reacting the sample with a condensed polycyclic compound and crystallizing the obtained molecules to be measured labeled with the condensed polycyclic compound and a matrix.
[5] The MALDI mass spectrometry method described in [1], wherein the multiple molecules to be measured are a glycopeptide mixture, saccharides and/or peptides of the glycopeptides may be different from each other, and a sample to be measured is prepared by reacting the sample with a condensed polycyclic compound, and crystallizing the obtained molecules to be measured labeled with the condensed polycyclic compound and a matrix.
[6] The MALDI mass spectrometry method described in any one of [3] to [5], wherein the condensed polycyclic compound is a pyrene ring compound.
[7] The MALDI mass spectrometry method described in [6], wherein the pyrene ring compound is 1-pyrenyldiazomethane (PDAM).
[8] The MALDI mass spectrometry method described in any one of [1] to [7], wherein the multiple molecules to be measured existing in the sample to be measured are each 10 pmol or less.

### EFFECTS OF THE INVENTION

According to the present invention, a measurement of the multiple molecules to be measured can be carried out quantitatively within a short period of time with good efficiency by removing the above-mentioned problems and solving the above-mentioned tasks.

More specifically, it can be provided a mass spectrometry method which can realize a quantitative measurement of the multiple molecules to be measured by measuring an arbitrary point which detects a signal of a part of the sample without measuring and integrated averaging the entire sample to be measured. Therefore, even when an amount of the sample containing molecules to be measured is extremely minute, reproducibility of the measurement can be improved, and information concerning chemical structures can be obtained with high reliability.

Further, in the case of a mixture of multiple molecules to be measured, there are possibilities that ionization efficiencies of the respective molecules to be measured are different from each other, or each inhibits ionization to each other, so that if the entire sample to be measured is integrated and averaged, quantitative results rather cannot be obtained. However, according to the present invention, the molecules to be measured of the multiple molecules to be measured in the mixture become to show the same ionization efficiency or signal distribution, so that if the entire sample to be measured is integrated and averaged or one arbitrary point which detects a signal is measured, a quantitative result can be obtained.

In addition, the present invention can be applied to molecules derived from a living body sample or molecules in a sample of a living body with a minute amount, so that, for example, it can analyze the chemical structure of the molecules such as a saccharide, a glycopeptide, a glycoprotein, etc., as a matter of course, and information useful for elucidation of the function or elucidation of pathological conditions can be more suitably obtained.

It can be also applied to biologics, so that an existing ratio of the respective glycoforms becomes clear, and it can be also applied to estimate a pharmacological activity or an adverse effect, or to quality control.

### BRIEF DESCRIPTION OF DRAWINGS

[FIG. 1]

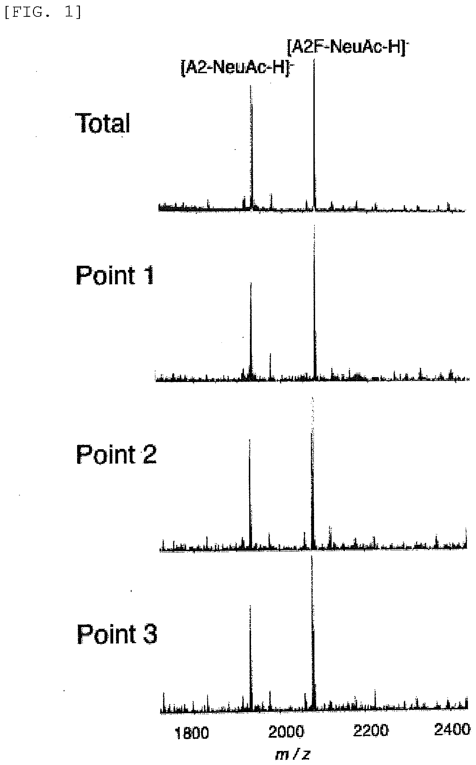

   FIG. 1 is a chart showing a MS spectrum of a sample including two kinds of saccharide (A2, A2F), as a molecule to be measured, labeled by a pyrene ring compound, in Example 1. "Total" shows a MS spectrum in which the entire sample to be measured was performed to an automatic measurement. "Points 1 to 3" show an individual MS spectrum at three points of the sample to be measured.
[FIG. 2]

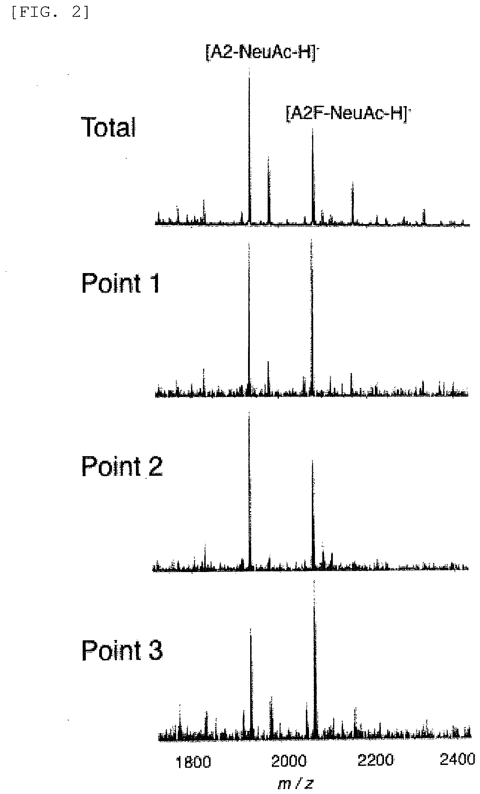

   FIG. 2 is a chart showing a MS spectrum of a sample to be measured including two kinds of saccharide (A2, A2F), as a molecule to be measured, in Comparative Example 1. "Total" shows a MS spectrum in which the entire sample to be measured was performed to an automatic measurement. "Points 1 to 3" show an individual MS spectrum at three points of the sample to be measured.
[FIG. 3]

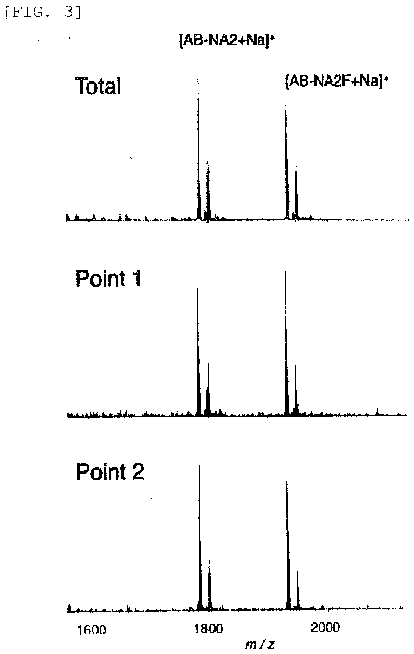

   FIG. 3 is a chart showing a MS spectrum of a sample including two kinds of saccharide (2AB-NA2, 2AB-NA2F), as a molecule to be measured, labeled by 2-aminobenzamide, in Comparative Example 2. "Total" shows a MS spectrum in which the entire sample to be measured was performed to an automatic measurement. "Points 1 to 2" show an individual MS spectrum at two points of the sample to be measured.
[FIG. 4]

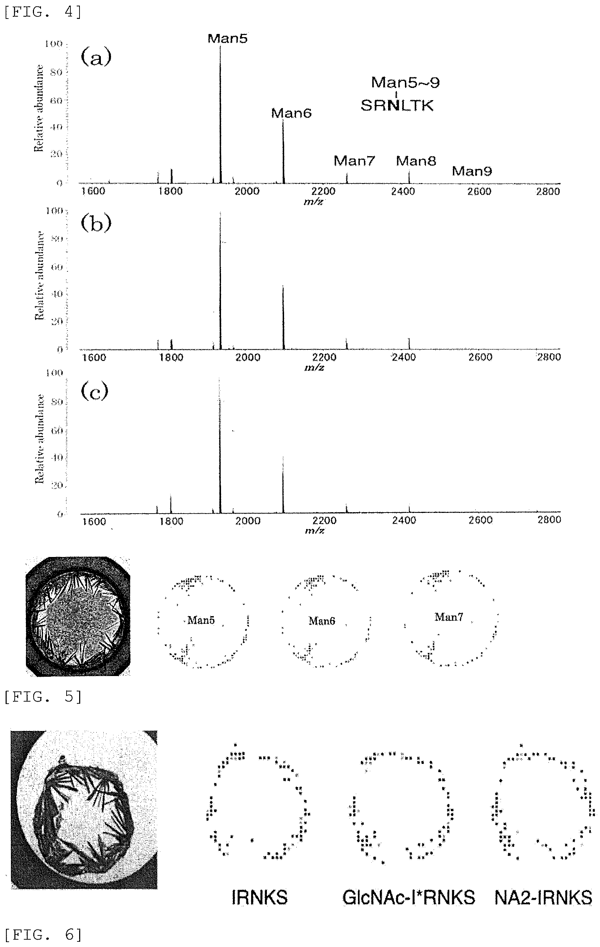

   FIG. 4 is a chart showing a MS spectrum of a sample including five kinds of glycopeptide as a molecule to be measured, a confocal laser microphotograph of a sample to be measured, and a chart showing a distribution of the points at which signals were detected of the molecule to be measured, in Example 2. "(a) to (c)" show an individual MS spectrum at three points of the sample to be measured.
[FIG. 5]
   FIG. 5 is a confocal laser microphotograph of a sample to be measured, and a chart showing a distribution of the points at which signals were detected of three kinds of a molecule to be measured, in Example 3.
[FIG. 6]
   FIG. 6 is a confocal laser microphotograph of a sample to be measured, and a chart showing a distribution of the points at which signals were detected of three kinds of a molecule to be measured, in Example 4.
[FIG. 7]

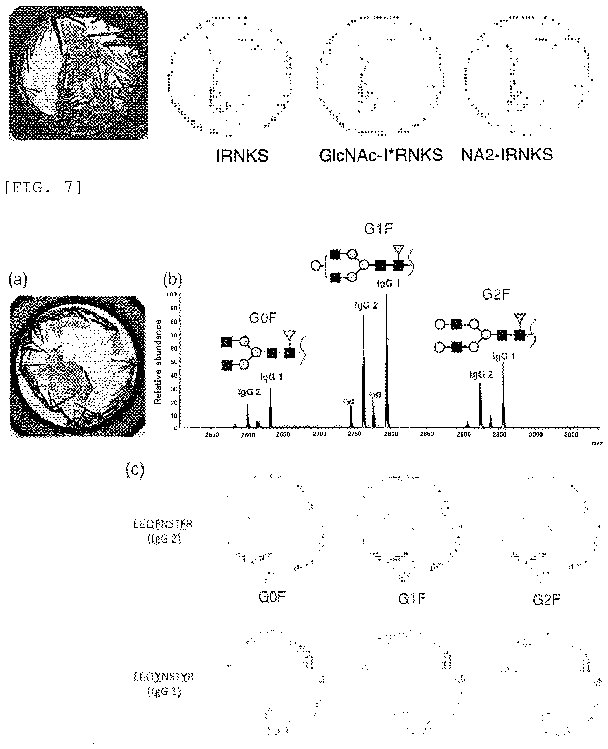

   FIG. 7 is a chart showing a MS spectrum of a sample including six kinds of glycopeptide as a molecule to be measured, a confocal laser microphotograph of the sample to be measured, and a chart showing a distribution of the points at which signals were detected of the molecule to be measured, in Example 5 and Comparative Example 3. "a" shows a confocal laser microphotograph of a sample to be measured. "b" shows a MS spectrum in which the entire sample to be measured was performed to an automatic measurement. "c" shows a distribution of the points at which signals of three kinds of glycopeptide having a structure of "EEQFNSTFR" as a peptide were detected, and a distribution of the points at which signals of three kinds of glycopeptide having a structure of "EEQYNSTYR" as a peptide were detected.
[FIG. 8]

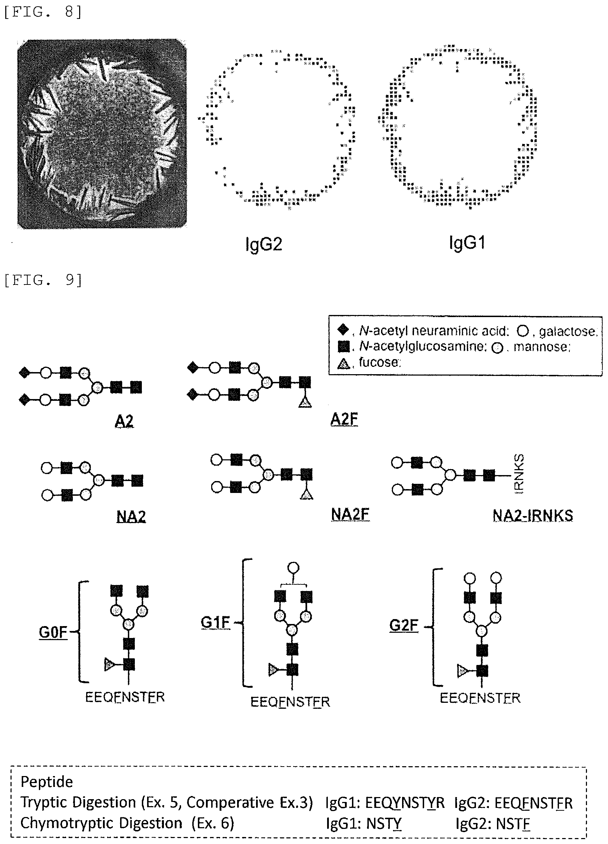

   FIG. 8 is a confocal laser microphotograph of a sample to be measured, and a chart showing a distribution of the points at which signals were detected of a molecule to be measured, in Example 6.
[FIG. 9]
   FIG. 9 is a chart showing structures used in Examples.
[FIG. 10]

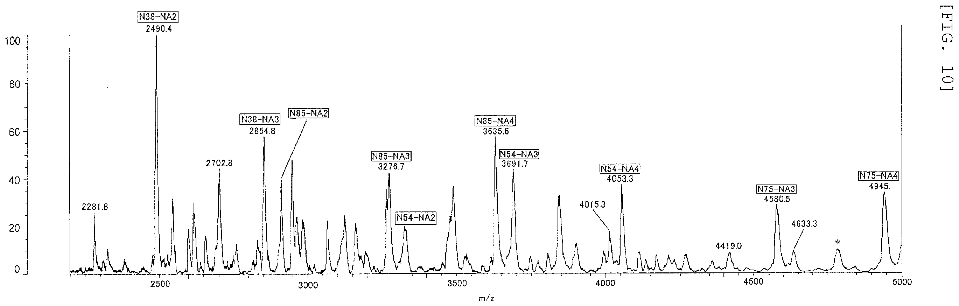

   FIG. 10 is a chart showing a mass spectrum of a Glu-C digested-glycopeptide mixture, in Example 8.
[FIG. 11]

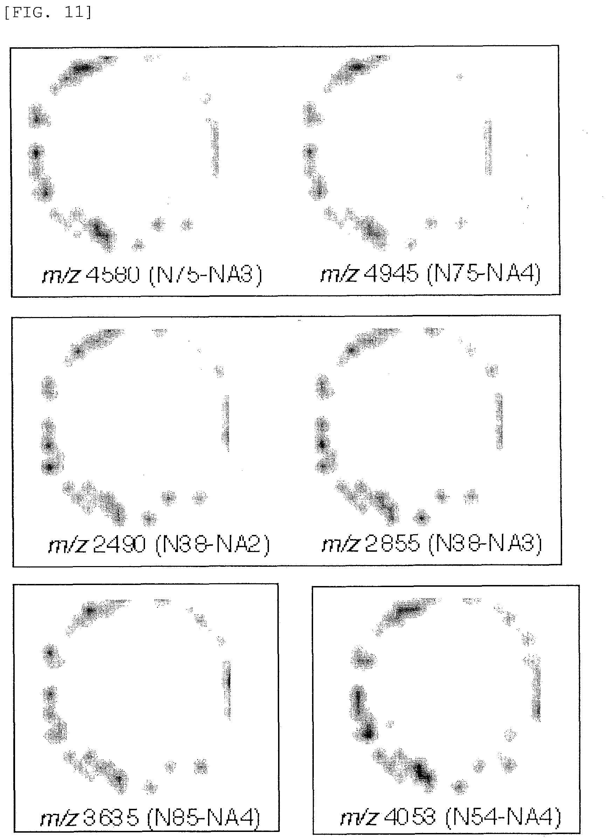

   FIG. 11 is a chart showing a distribution of the points at which signals were detected of a molecule to be measured, in Example 8.
[FIG. 12]

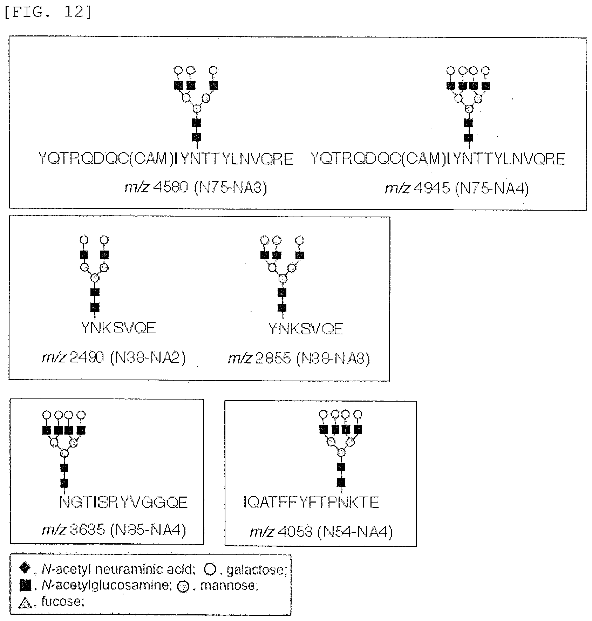

   FIG. 12 is a chart showing structures used in Examples.

### EMBODIMENTS TO CARRY OUT THE INVENTION

The MALDI mass spectrometry method of the present invention is a mass spectrometry method using a sample to be measured for the mass spectrometry method (in the present invention, it is sometimes abbreviated simply to as "sample to be measured") prepared from a sample containing multiple molecules to be measured and a matrix, and the multiple molecules to be measured have a common peptide structure or a common derivatized structure by a derivatizing agent so that a point which detects a signal is formed at the common position, whereby a quantitative mass spectrum can be obtained by only irradiating a laser onto any measurement point of the sample to be measured to measure an individual spectrum without integrated averaging the spectra obtained by repeating laser shots, for example, several hundreds of times or more over the entire sample to be measured.

The MALDI mass spectrometry method of the present invention is a MALDI mass spectrometry method for a sample containing multiple molecules to be measured, characterized in that the multiple molecules to be measured are a saccharide mixture, a glycopeptide mixture, a glycopeptide-peptide mixture, a glycoprotein mixture, or a glycoprotein-protein mixture, and a quantitative mass spectrum of the multiple molecules to be measured is obtained from an arbitrary point in the sample to be measured where a signal is detected without measuring and integrated averaging the entire sample to be measured.

### <Molecules to be measured>

The molecules to be measured to which the MALDI mass spectrometry method of the present invention is applied are a saccharide, a glycopeptide, a peptide, a glycoprotein or a protein, particularly preferably a saccharide, a glycopeptide or a glycoprotein.

In the present invention, the terms "a saccharide" refer mainly to a glycan (an oligosaccharide, a polysaccharide), and refer to whole saccharides including a monosaccharide. It also contains a glycoprotein and "a saccharide" derived from a glycolipid.

The "molecules to be measured" may include a material prepared from a natural product and a material prepared by chemically or enzymatically modifying a part of a natural product, and also a chemically or enzymatically prepared material is preferred. In addition, a material having a partial structure of the molecule contained in a living body or a material prepared by imitating a molecule contained in a living body is also preferred.

A sample mounting on a sample-supporting member (in the following, it is sometimes abbreviated to as a "plate") to be used in the mass spectrometry method, i.e., a sample containing the molecules to be measured may be mentioned the "molecules to be measured" itself alone, or may be a material containing the "molecules to be measured".

Also, the sample may be those obtained from, for example, a tissue, cells, a body fluid or a secretion (for example, a blood, serum, urine, semen, saliva, tears, sweat, feces, etc.) of a living body, and the like.

The molecules to be measured may be prepared by mounting a sample on a plate, and subjecting to enzymatic treatment, etc.

In "the sample containing the multiple molecules to be measured" may accordingly contain molecules other than the molecules to be measured, and in "the molecules other than the molecules to be measured" herein mentioned, a saccharide, a glycopeptide, a peptide, a glycoprotein, a protein, a glycolipid, a lipid, etc., all of which are not an object to be measured, may be contained.

The above-mentioned molecules are in many cases provided with a little amount of the sample to be applied to the analysis. In particular, a saccharide, a glycoconjugate such as a glycopeptide and a glycoprotein, or a material obtained by chemically or enzymatically liberating therefrom is extremely poor in efficiency of ionization at the saccharide portion, or a point(s) of detecting a signal including a sweet spot is difficultly found out so that the measurement was gave up in many cases. The MALDI mass spectrometry method of the present invention is preferred since it accomplishes the above-mentioned remarkable effects particularly on the analysis of the chemical structures of those molecules.

In the MALDI mass spectrometry method of the present invention, the above-mentioned "multiple molecules to be measured" is a saccharide mixture, a glycopeptide mixture, a glycopeptide-peptide mixture, a glycoprotein mixture, or a glycoprotein-protein mixture.

The MALDI mass spectrometry method of the present invention is preferred in the points that:
(a) the multiple molecules to be measured are a glycopeptide mixture, and the peptide portions of which are the same with each other, or,
(b) the multiple molecules to be measured are a glycopeptide-peptide mixture, and the peptide portions of glycopeptide are the same with each other, and the peptide portion of the glycopeptide and the peptide are the same with each other,
since the points which detect the signals become common with regard to the multiple molecules to be measured in "a mixed crystal or a mixture of a sample and a matrix" which is the sample to be measured and prepared on a sample-supporting member, so that if a laser is irradiated to an arbitrary point which detects a signal of the sample to be measured, quantitative mass spectra can be obtained with regard to the multiple molecules to be measured. That is, it is preferred in the point that a contained ratio of the respective molecules of the multiple molecules to be measured in the sample can be accurately obtained and grasped when a laser is irradiated to a certain point(s) onto the sample to be measured.

Also, the MALDI mass spectrometry method of the present invention is preferred in the point that:
(c) the multiple molecules to be measured are a saccharide mixture, and a sample to be measured is prepared by reacting the sample with a condensed polycyclic compound, and crystallizing the obtained molecules to be measured labeled with the condensed polycyclic compound and a matrix,
since the points which detect the signals become common with regard to the multiple molecules to be measured in "a mixed crystal or a mixture of a sample and a matrix" which is the sample to be measured and prepared on a sample-supporting member, so that if a laser is irradiated to the point(s) which detect the signal of the sample to be measured, quantitative mass spectra can be obtained with regard to the multiple molecules to be measured. That is, it is preferred in the point that a contained ratio of the respective molecules of the multiple molecules to be measured in the sample can be accurately obtained and grasped when a laser is irradiated to a certain point(s) onto the sample to be measured.

Also, the MALDI mass spectrometry method of the present invention is preferred in the point that:
(bc) the multiple molecules to be measured are a glycopeptide-peptide mixture, the peptide portions of the glycopeptides are the same with each other, the peptide portion of the glycopeptide and the peptide are the same with each other, and a sample to be measured is prepared by reacting the sample with a condensed polycyclic compound, and crystallizing the obtained molecules to be measured labeled with the condensed polycyclic compound and a matrix,
since the points which detect the signals become common with regard to the multiple molecules to be measured in "a mixed crystal or a mixture of a sample and a matrix" which is the sample to be measured and prepared on a sample-supporting member, so that if a laser is irradiated to the point(s) which detect the signal of the sample to be measured, quantitative mass spectra can be obtained with regard to the multiple molecules to be measured. That is, it is preferred in the point that a contained ratio of the respective molecules of the multiple molecules to be measured in the sample can be accurately obtained and grasped when a laser is irradiated to a certain point(s) onto the sample to be measured.

Further, the MALDI mass spectrometry method of the present invention is preferred in the point that:
(c') the multiple molecules to be measured are a glycopeptide mixture, saccharides and/or peptides of the glycopeptides may be different from each other, and a sample to be measured is prepared by reacting the sample with a condensed polycyclic compound, and crystallizing the obtained molecules to be measured labeled with the condensed polycyclic compound and a matrix,
since the points which detect the signals become common with regard to the multiple molecules to be measured in "a mixed crystal or a mixture of a sample and a matrix" which is the sample to be measured and prepared on a sample-supporting member, so that if a laser is irradiated to the point(s) which detect the signal of the sample to be measured, quantitative mass spectra can be obtained with regard to the multiple molecules to be measured. That is, it is preferred in the point that a contained ratio of the respective molecules of the multiple molecules to be measured in the sample can be accurately obtained and grasped when a laser is irradiated to a certain point(s) onto the sample to be measured.

It has conventionally been known a method of obtaining a mass spectrum of multiple molecules to be measured from one arbitrary point which detects a signal of a sample to be measured, without measuring and integrated averaging the entire sample to be measured.

However, if a person wishes to know the chemical structure at the glycan portion of a glycopeptide (a glycoprotein), it has generally been carried out to prepare a sample to be measured by removing the peptide portion (the protein portion), or to measure and integrated average the entire sample to be measured (integration of multipoint measurements has been done) without removing the same, so that it was not an effective method.

The invention of the present application has been done by finding out that, even when the peptide portion (the protein portion) is not removed, and further, in "the specific combination of the molecules to be measured or the specific form of the sample to be measured" like (a) or (b); (c); (bc); (c') as mentioned above, when it is rather not removed, a point(s) which detects a signal with regard to the multiple molecules to be measured becomes common, whereby the above-mentioned remarkable effects can be consequently obtained.

It has also conventionally been known a method of obtaining a mass spectrum of the multiple molecules to be measured from one arbitrary point which detects a signal of a sample to be measured by labeling the molecules to be measured with a derivatizing agent without measuring and integrated averaging the obtained entire sample to be measured.

However, when the molecules to be measured is labeled with a specific derivatizing agent, irrespective of removing or without removing the peptide portion (the protein portion), "a point which detects a signal" including a sweet spot becomes common with regard to the multiple molecules to be measured, and consequently, the inventors have found out that the above-mentioned remarkable effects can be obtained whereby the invention of the present application has been accomplished.

When the molecules to be measured is a peptide or a glycopeptide, the peptide or the glycopeptide is preferably a material in which a protein or a glycoprotein is chemically fragmented, or prepared by using an enzyme such as trypsin, thermolysin, chymotrypsin, endoproteinase Lys-C, endoproteinase Arg-C, endoproteinase Glu-C, etc., singly or in combination. Whether which method is the optimum or not may vary depending on an amino acid sequence of the protein, and if a smaller sized peptide is to be obtained, thermolysin, etc., are preferably used, while the same C-terminal amino acid is to be obtained, endoproteinase Lys-C, endoproteinase Arg-C, endoproteinase Glu-C, etc., are preferably used.

When PDAM is used as the derivatizing agent, a carboxyl group at the C-terminal amino acid is present in any of the above-mentioned methods so that it is suitable, and when endoproteinase Glu-C is used, a carboxyl group is also present at the C-terminal amino acid side chain so that it is particularly preferred.

### <Measurement point>

The MALDI mass spectrometry method of the present invention is characterized in that a quantitative mass spectrum for the multiple molecules to be measured can be obtained from an arbitrary point which detects a signal of the sample to be measured, which is not necessarily a sweet spot, without measuring and integrated averaging the entire sample to be measured.

In the sample to be measured including the "multiple molecules to be measured" which is extremely limited in the present invention, when a laser is irradiated to a point(s) which detects a signal onto the sample to be measured, the point(s) is common in the "multiple molecules to be measured", so that a contained ratio of the respective molecules in the sample containing the multiple molecules to be measured can be accurately obtained and grasped.

The "an arbitrary point which detects a signal of the sample to be measured" includes the so-called sweet spot, but it may be an arbitrary point so long as a signal can be obtained, which is not necessarily a point with high sensitivity, so that it is not limited to the "sweet spot".

The "sweet spot" means a place from which an ion derived from the molecules to be measured can be obtained with particularly high sensitivity when the laser is irradiated to the place.

A size of "the point(s) which detects a signal of the sample to be measured" generally equals to an irradiation area of the laser at the one place, and preferably an irradiation area of the laser at the one place of the device for a commercially available MALDI mass spectrometry method. More specifically, it is preferably 10,000 µm² (100 µm×100 µm) or less, particularly preferably 100 µm² (10 µm×10 µm) to 2,500 µm² (50 µm×50 µm).

It is essential in the present invention that the "quantitative mass spectrum for the multiple molecules to be measured" is obtained from at least one arbitrary point (including a sweet spot) which detects a signal of the molecules to be measured, but "quantitative mass spectra for the multiple molecules to be measured" may be obtained from several points which are two or more arbitrary points which detect a signal of the sample to be measured, and these may be averaged. However, it is excluded to measure and integrated average the entire sample to be measured.

The number of the laser shots to one arbitrary point which detects a signal is not limited, and preferably 5 to 20 shots.

### <Precipitation of mixed crystal or formation of mixture of sample and matrix>

In the present invention, a mixed crystal of a sample containing molecules to be measured and a matrix in the MALDI mass spectrometry method is firstly precipitated. The method for precipitating the mixed crystal is not particularly limited, and as preferred methods, for example, the following (1) to (3) are mentioned.
(1) A method of precipitating a mixed crystal by placing a sample containing molecules to be measured onto a sample-supporting member, placing a solution of a matrix thereon after drying or before drying, then, drying the solution.
(2) A method of precipitating a mixed crystal by simultaneously placing a sample containing molecules to be measured and a solution of a matrix onto a sample-supporting member, then, drying the solution.
(3) A method of forming a mixture by placing a solution of a matrix onto a sample-supporting member, then, after drying the solution to precipitate a crystal of the matrix, placing a solution containing a matrix having the same or different chemical structures and molecules to be measured over the crystal, then, drying the solution.

In the case of a liquid matrix, the procedure is, for example, as follows.
(1) A method of forming a mixture by placing a sample containing molecules to be measured onto a sample-supporting member, placing a solution of a matrix thereon after drying or before drying, then, evaporating the solvent of the mixed solution.
(2) A method of forming a mixture by simultaneously placing a sample containing molecules to be measured and a solution of a matrix onto a sample-supporting member, then, evaporating the solvent of the mixed solution.

The method for precipitating a mixed crystal of a sample and a matrix is particularly preferably the above-mentioned (1) or (2). That is, it is a method in which
(1) after placing a sample containing molecules to be measured onto a sample-supporting member, placing a solution of a matrix, or,
(2) simultaneously placing a sample containing molecules to be measured and a solution of a matrix onto the sample-supporting member,
then, drying the solution to precipitate the mixed crystal.

### <<Method (1)>>

The above-mentioned Method (1) is a method particularly suitable in the present invention, and is a method for preparing a sample to be measured in which a sample containing molecules to be measured is previously presented onto a sample-supporting member, then, a solution of a matrix (preferably a solution containing substantially the matrix alone) is layered over the sample, and dried on the sample-supporting member (in the following, it is sometimes abbreviated to as a "plate") while dissolving the sample, whereby the crystal is precipitated.

In the above-mentioned (1), the step of drying the matrix solution on the plate is made the final step among the steps of supplying the material onto the plate. In general, it has been said that for obtaining a high ion formation amount, the sample and the matrix must be mixed well, and if the above-mentioned method of (1) is used, even if the molecules to be measured is not dissolved (contained) in the solution to be supplied onto the plate at the final step, it is mixed well with the matrix.

When a solution comprising the matrix alone is used at substantially the final step for mounting a material onto a plate, the solvent of the solution is preferably a solvent which can dissolve both of the molecules to be measured and the matrix. This is because it can dissolve the molecules to be measured which have already been presented at the under layer and can mix with the matrix.

The above-mentioned Method (1) preferably contains the following Step (A) and Step (B) as essential steps.
(A) a step of drying a solution of a sample containing molecules to be measured on a plate, and then,
(B) a step of dropping a solution in which a matrix alone is dissolved in a solvent onto the plate.

In Step (A), the solution of the sample containing the molecules to be measured is dried onto the plate. The solution at this time may be any solution so long as it contains the molecules to be measured, and the matrix may be contained or may not be contained. However, in the case that an amount of the sample containing the molecules to be measured is extremely minute, when a good solvent of the sample containing the molecules to be measured is a poor solvent of the matrix, and the matrix or the solvent is desired to be used, or when the sample is unstable to the solvent in which the matrix is dissolved and it cannot be stored or used as a stock solution or difficultly handled, etc., then, the solution in such a case preferably be a solution in which sample containing the molecules to be measured alone is dissolved and a matrix is not dissolved therein in the points that preparation of the sample to be measured is easy, choices of the solvent are broadened, decomposition of the sample or adsorption of the same to a container can be suppressed, etc.

The solvent at that time is not particularly limited so long as it can dissolve the sample containing molecules to be measured, and it may not necessarily have a property to dissolve the matrix, or it may have a high boiling point at normal pressure, and an evaporation (drying) rate thereof may be late. When the molecules to be measured or the sample containing the molecules to be measured is water-soluble, the solvent to be used in Step (A) is particularly preferably water alone. When the sample containing the molecules to be measured is a saccharide, the solvent to be used in Step (A) is most preferably water alone. It does not dissolve the matrix, so that the solubility to the matrix may be low, whereby the most suitable solvent to the molecules to be measured or the sample containing the molecules to be measured can be selected.

A concentration of the molecules to be measured in the solution in Step (A) is not particularly limited, and is preferably from 1 amol/µL to 1 µmol/µL, particularly preferably from 100 amol/µL to 1 nmol/µL. A thickness of the layer containing the molecules to be measured existing on a plate after completion of Step (A) (after drying) is not particularly limited, and is preferably 5 µm or less, particularly preferably 1 µm or less. If the thickness of such a layer is too thick, when the matrix solution is dropped onto the layer, there is a possibility that the sample is not mixed with the matrix molecule well, so that the MS signal intensity is sometimes lowered.

In Method (1), since a solution of the "sample containing molecules to be measured" is firstly dropped onto the plate, when an amount of the sample containing molecules to be measured is extremely minute, almost all the amount of the minute amount sample can be applied to the analysis, without mixing it with a matrix solution in a separate container and a part thereof is dropped onto the plate, it is particularly useful.

In Method (1), the sample contains the multiple molecules to be measured, and the respective molecules to be measured are preferably each 10 pmol or less, more preferably 1 pmol (1,000 fmol) or less, particularly preferably 300 fmol or less, and 100 fmol (for example, by dropping 1 µL of an aqueous solution in which the respective molecules to be measured are dissolved each in an amount of 100 fmol/µL, etc., to place 100 fmol thereof onto the plate, etc.) or less is more useful.

In Method (1), after Step (A), it has (B) a step of dropping a solution in which a matrix alone is dissolved in a solvent is dropped onto the above-mentioned plate. The meaning of "after Step (A)" is not limited to "immediately after Step (A)", and other step(s) may be inserted between Step (A) and Step (B). As the "other step(s)" may be mentioned, for example, Step (C) mentioned later.

In Step (B), while a solution in which a matrix alone is substantially dissolved in a solvent is dropped onto the above-mentioned plate, by using a solvent which can dissolve both of the molecules to be measured and the matrix, the molecules to be measured at the under layer is mixed with the matrix molecule, and after drying, a mixed crystal is appeared to form a sweet spot.

### <<<Derivatization>>>

In Method (1), when derivatization of the sample to be measured is carried out, between the above Step (A) and Step (B),
(C) a step of dropping a solution of a derivatizing agent which heightens ionization efficiency by reacting with the molecules to be measured onto the above-mentioned plate and drying the same is preferably contained to further heighten ionization efficiency.

That is, the MALDI mass spectrometry method in which after (A) placing the sample containing molecules to be measured onto the sample-supporting member, and before (B) placing the solution of the matrix, (C) a step of dropping the solution of the derivatizing agent which heightens ionization efficiency by reacting with the molecules to be measured onto the sample-supporting member and drying the same is inserted, is preferred since ionization efficiency can be further heightened.

When the derivatizing agent (in the following, it is simply abbreviating to as "derivatizing agent") which heightens ionization efficiency by reacting with the molecules to be measured is supplied onto the plate after previously reacting with the molecules to be measured, there is a case where loss of the sample to be measured is generated. Accordingly, when the mass spectrometry method is applied to the "molecules derived from a living body or molecules in a sample of a living body" with a small amount such as a saccharide, a glycoprotein, a glycopeptide, etc., it is particularly preferred to insert Step (C) between Step (A) and Step (B).

The derivatizing agent is not particularly limited so long as it heightens ionization efficiency of the derivatized molecules to be measured, i.e., the molecule to be applied to the mass spectrometry. The derivatizing agent is preferably a compound having an effect as a matrix in the MALDI mass spectrometry method, or a compound further having a reactive functional group or a spacer portion to the above mentioned later.

The chemical structure of such a derivatizing agent is a condensed polycyclic compound having a condensed polycyclic ring in the molecule such as naphthalene, anthracene, pyrene, etc., since they suitably accomplish the above-mentioned effect (an effect of heightening ionization efficiency), and yet a point on the sample to be measured which detects a signal of the molecules to be measured becomes common on the sample-supporting member. When a laser is irradiated onto "an arbitrary point of the sample to be measured which detects a signal", a quantitative mass spectrum which reflects a mixing ratio in the sample can be obtained with regard to the multiple molecules to be measured.

Here, "the condensed polycyclic compound" refers to a compound having a reactive functional group capable of bonding a condensed polycyclic portion which may contain a heterocyclic ring containing a nitrogen, sulfur or oxygen molecule at a part thereof, and molecules to be measured, and, if necessary, a spacer portion which links the condensed polycyclic portion and the reactive functional group. In particular, it is preferably a condensed polycyclic aromatic compound having an aromatic ring.

The derivatizing agent is particularly preferably a material which can control the ionization cutting position of the derivatized molecule, i.e., the molecule to be supplied to the mass spectrometry by reacting with the molecules to be measured.

The derivatizing agent preferably has a reactive functional group such as an amino group, a hydrazide group, a diazomethyl group, a succinimidyl ester group, a sulfonyl chloride group, an iodo group (-I), etc. Particularly preferred derivatizing agent may be specifically mentioned a condensed polycyclic derivative compound in which the above-mentioned group(s) is/are bonded directly or through the other group (a spacer portion) to a condensed polycyclic such as a naphthalene ring, an anthracene ring, a pyrene ring, etc.; methyl iodide; diazomethane; trimethylsilyldiazomethane, etc.

Among these, the pyrene ring compound is particularly preferred in the points that ionization efficiency of the derivatized molecule, i.e., the molecule to be applied to the mass spectrometry is heightened; ionization cutting position can be controlled; "a point(s) which detects a signal of a sample to be measured" becomes common with regard to the multiple molecules to be measured on the prepared sample to be measured onto the sample-supporting member; etc.

Here, "the pyrene ring compound" refers to a compound having a pyrene ring, a reactive functional group capable of bonding to "the molecules to be measured", and, if necessary, a spacer portion which links the pyrene ring and the reactive functional group.

More specifically, 1-pyrenebutanoic acid hydrazide (in the following, it is abbreviated to as "PBH"), 1-pyreneacetic acid hydrazide, 1-pyrenepropionic acid hydrazide, 1-pyreneacetic acid succinimidyl ester, 1-pyrenepropionic acid succinimidyl ester, 1-pyrenebutanoic acid succinimidyl ester, N-(1-pyrenebutanoyl)cysteic acid succinimidyl ester, N-(1-pyrene)iodoacetamide, N-(1-pyrene)maleimide, N-(1-pyrenemethyl)iodoacetamide, 1-pyrenemethyl iodoacetate, aminopyrene, 1-pyrenemethyl amine, 3-(1-pyrenyl)propylamine, 4-(1-pyrenyl)butylamine, 1-pyrenesulfonyl chloride, 1-pyrenyldiazomethane (in the following, it is abbreviated to as "PDAM"), 1-pyrenecarbaldehyde hydrazone, 1-pyrenylthiocyanate, 1-pyrenylisothiocyanate, etc., are preferred.

In the specific compounds, a naphthalene ring, or an anthracene ring instead of a pyrene ring is preferred, as a derivatizing agent. Further, methyl iodide, diazomethane, or trimethylsilyldiazomethane is preferred.

Among these, in the sample to be measured prepared onto the sample-supporting member, "a point(s) which detects a signal" becomes common with regard to the multiple molecules to be measured, more preferred is PBH or PDAM, and most preferred is PDAM. If a laser is irradiated to "an arbitrary point of a sample to be measured which detects a signal", a quantitative mass spectrum can be obtained with good efficiency with regard to the multiple molecules to be measured.

Preferred "combination of the molecules to be measured and the derivatizing agent" may be mentioned the case where the molecules to be measured are molecules having a glycan containing an aldehyde group, and the derivatizing agent is a material having an amino group or a hydrazide group, etc.

Also, preferred combination may be mentioned the case where the molecules to be measured are a glycopeptide or a peptide having a carboxyl group, an amino group or a mercapto group, or a glycoprotein or a protein having such groups, and the derivatizing agent is a material having an amino group, a hydrazide group or a diazomethyl group, etc., and further the case where the molecules to be measured are a glycopeptide or a peptide having a carboxyl group, or a glycoprotein or a protein having such groups, and the derivatizing agent is methyl iodide or trimethylsilyldiazomethane.

These combinations are preferred in the points that the molecules to be measured and the derivatizing agent can be easily reacted on a plate, ionization is not inhibited, the reaction is selective, and the above-mentioned effects can be easily accomplished since necessity to analyze with a minute amount is generally high, etc.

A reaction temperature of the molecules to be measured and the derivatizing agent is not particularly limited, and the reaction is preferably carried out at 40 to 100°C, particularly preferably carried out at 60 to 90°C. By carrying out the reaction at the temperature higher than the room temperature, the reaction rate is increased.

### <<Method (2)>>

It is preferred (2) the method in which solutions of the sample containing molecules to be measured and a solution of the matrix are simultaneously placed onto the sample-supporting member, the solution is dried to precipitate the mixed crystal onto the sample-supporting member. Such a preparation method of a sample to be measured of (2) has been known as the "Dried Droplet method". A method in which after placing the sample containing the molecules to be measured onto the sample-supporting member according to the preparation method of (1), a solution of a matrix is placed before drying is the same.

In Method (2), the sample contains multiple molecules to be measured, and the respective molecules to be measured are each preferably the case of 10 pmol or less, more preferably the case of 1 pmol (1,000 fmol) or less, particularly preferably the case of 300 fmol or less, and further preferably the case of 100 fmol or less.

In this Method (2), derivatization of the molecules to be measured may be carried out. "Preferred derivatizing agent", etc., are the same as those of Method (1).

### <Matrix solution>

A matrix is not limited if the matrix is a material achieving desorption and ionization of molecules to be analyzed which coexist by absorbing light energy of a laser, irrespective of liquid or solid, and the following compounds are examples.

1,8-Diaminonaphthalene (1,8-DAN), 2,5-Dihydroxybenzoic acid (in the following, it is abbreviated to as "DHBA"), 1,8-Anthracenedicarboxylic Acid Dimethyl ester, Leucoquinizarin, Anthrarobin, 1,5-Diaminonaphthalene (1,5-DAN), 6-Aza-2-thiothymine, 1,5-Diaminoanthraquinone, 1,6-Diaminopyrene, 3,6-Diaminocarbazole, 1,8-Anthracenedicarboxylic Acid, Norharmane, 1-Pyrenepropylamine hydrochloride, 9-Aminofluorene Hydrochloride, Ferulic acid, Dithranol, 2-(4-Hydroxyphenylazo) benzoic acid (HABA), trans-2-[3-(4-tert-Butylphenyl)-2-methyl-2-propenylidene]malononitrile) (DCTB), trans-4-Phenyl-3-buten-2-one) (TPBO), trans-3-Indoleacrylic acid (IAA), 1,10-Phenanthroline, 5-Nitro-1,10-phenanthroline, α-Cyano-4-hydroxycinnamic acid (CHCA), Sinapic acid (SA), 2,4,6-Trihydroxyacetophenone (THAP), 3-Hydroxypicolinic acid (HPA), Anthranilic acid, Nicotinic acid, 3-Aminoquinoline, 2-Hydroxy-5-methoxybenzoic acid, 2,5-Dimethoxybenzoic acid, 4,7-Phenanthroline, p-Coumaric acid, 1-Isoquinolinol, 2-Picolinic acid, 1-Pyrenebutanoic acid hydrazide (PBH), 1-Pyrenebutyric acid (PBA), 1-Pyrenemethylamine hydrochloride (PMA), 3-AC(aminoquinoline)-CHCA.

A solution for dissolving a matrix is not limited, and water, acetonitrile, methanol, ethanol, and a mixed solution thereof are examples.

A dry method of a matrix solution is especially limited, and promotion of drying by heating, cooling, blasting, decompressing, etc. may be effective.

### <Mass spectrometer>

A nitrogen laser (337 nm), a YAG laser triple wave (355 nm), an NdYAG laser (256 nm), a carbon dioxide laser (2940 nm), etc., are examples as a laser used for ionization, a nitrogen laser is preferred. A method of separating and detecting an ion is not limited, a double focusing method, a quadrupole focusing method (quadrupole (Q) filter method), a tandem quadrupole (QQ) method, an ion trap method, or a time-of-flight (TOF) method, etc., is used for separating and detecting an ionized ion according to a mass-to-charge ratio (m/z). QIT-TOF is preferred.

Since a molecule of a saccharide, a glycoprotein, a glycopeptide, and a glycolipid, etc., contains many isomers which have the same molecular weight and composition, a method of repeating fragmentation of a molecule at n time (MSⁿ method) so as to improve the product efficiency of an ion is preferred. An MSⁿ method can determine a binding position in a molecule, for example.

### EXAMPLES

### Example 1

### <A2/A2F mixture, PDAM labeled (derivatized), spectrum is constant irrespective of the measured points>

First, onto a plate (sample-supporting member) for mass spectrometry was dropped 1 µL of an aqueous solution which had been prepared by dissolving glycans A2 and A2F (respective chemical structures are shown in Fig. 9) which are molecules to be measured in water and each making the concentration 100 fmol/µL, and the plate was allowed to stand at room temperature (23°C) under atmospheric pressure to dry it.

Next, 0.25 µL of a solution which had been prepared by dissolving a labeling reagent PDAM (1-pyrenyldiazo-methan; available from Molecular Probes Inc.) which is a derivatizing agent in DMSO (dimethyl sulfoxide; available from SIGMA Co.), and making the concentration 10 nmol/µL was dropped onto the above, and the plate was allowed to stand on a heat block at 70°C under atmospheric pressure to dry it. To remove excess derivatizing agent, the plate was dipped in xylene (available from SIGMA Co.), whereby excess PDAM was removed and the plate was sufficiently dried.

Subsequently, 1.00 µL of an aqueous solution which had been prepared by dissolving high purity DHBA (available from Shimadzu Biotech) as a matrix in 60% aqueous acetonitrile solution, and making the concentration 10 mg/mL was dropped on a sample-supporting member, and the solvent was evaporated at room temperature under atmospheric pressure to crystallize it.

With regard to the sample to be measured, a mass spectrum was obtained by the MALDI mass spectrometry method. The measurement was carried out by using MALDI-QIT-TOF type mass spectrometer (AXIMA-QIT, available from Shimadzu Biotech) as a mass spectrometer, and under a negative ion mode.

Also, after the laser power was optimized to the threshold at which a signal of ions of the molecules to be measured is started to detect to the entire sample to be measured, irradiation of the laser was carried out with an interval of 50 µm to perform an automatic measurement.

The automatic measurement was so performed that 10 shots of laser were irradiated per one point, 2209 points were measured per one sample to be measured, and integrated and averaged spectrum was obtained.

The obtained mass spectrum was shown in Fig. 1. At the "Total" of Fig. 1, a spectrum in which the entire sample to be measured was performed to an automatic measurement and integrated and averaged was shown, and at the "Points 1 to 3", spectra obtained from different three points among those in which signals of the glycan had been markedly detected when a laser had been irradiated to the sample to be measured were shown.

### Comparative Example 1

### <A2/A2F mixture, not labeled (derivatized), signal patterns are different from each other depending on the measured points>

First, onto a plate (sample-supporting member) for mass spectrometry was dropped 1 µL of an aqueous solution which had been prepared by dissolving glycans A2 and A2F (chemical structures are shown in Fig. 9) which are molecules to be measured in water and making concentrations 2 pmol/µL and 1 pmol/µL, respectively, and the plate was allowed to stand at room temperature (23°C) under atmospheric pressure to dry it.

With regard to the sample to be measured, crystallization was carried out in the same manner as in Example 1, and a mass spectrum was obtained by the MALDI mass spectrometry method.

The obtained mass spectrum was shown in Fig. 2. At the "Total" of Fig. 2, a spectrum in which the entire sample to be measured was performed to an automatic measurement and integrated and averaged was shown, and at the "Points 1 to 3", spectra obtained from different three points among those in which signals of the glycan had been markedly detected when a laser had been irradiated to the sample to be measured were shown.

Comparative Example 2 <NA2/NA2F mixture, 2AB labeled (derivatized), signal patterns are different from each other depending on the measured points>

First, onto a plate (sample-supporting member) for mass spectrometry was dropped 1 µL of an aqueous solution which had been prepared by dissolving 2-aminobenzamide (in the following, it is abbreviated to as "2AB") derivatives of glycans NA2 and NA2F (chemical structures are shown in Fig. 9) which are molecules to be measured in water and making each concentration 100 fmol/µL, and the plate was allowed to stand at room temperature (23°C) under atmospheric pressure to dry it.

In the following, those labeled (derivatized) with 2AB are written as "2AB-NA2", "2AB-NA2F", etc.

With regard to the sample to be measured, crystallization was carried out in the same manner as in Example 1, and a mass spectrum in a positive ion mode was obtained by the MALDI mass spectrometry method.

The obtained mass spectrum was shown in Fig. 3. At the "Total" of Fig. 3, a spectrum in which the entire sample to be measured was performed to an automatic measurement, and integrated and averaged was shown, and at the "Points 1 to 2", spectra obtained from different two points among those in which signals of the glycan had been markedly detected when a laser had been irradiated to the sample to be measured were shown.

### <Results of Example 1, Comparative Example 1 and Comparative Example 2>

When the mass spectra of "Points 1 to 3" of Example 1 are compared to each other, relative intensities of the signals of two molecules to be measured are the constant irrespective of the places to which a laser was irradiated, no fluctuation in the same sample to be measured, and each spectrum at the respective points matched with the spectrum obtained by automatically measuring and integrated averaging the entire sample to be measured.

On the other hand, when the mass spectra of "Points 1 to 3" in Fig. 2 of Comparative Example 1 are compared to each other, it is clear that relative intensities of the signals of two molecules to be measured are different from each other with the places to which a laser was irradiated, and there are fluctuation in the same sample to be measured. More specifically, Point 2 showed a similar spectrum to the average spectrum of the entire sample, but in Point 1 and Point 3, the intensity ratio of the two signals was reversed to that of the average spectrum.

Further, also in Comparative Example 2, when the spectra at Point 1 and Point 2 were compared to each other, the intensity ratio of the two signals was different from each other depending on the places to be measured.

This means that, in the unlabeled saccharide (Comparative Example 1) or the saccharide (Comparative Example 2) derivatized by 2-AB whereas which became high sensitivity as compared with the unlabeled saccharide, a relative intensity of each signal was different from each other depending on the places to be measured, but in the saccharide (Example 1) derivatized (labeled) by using PDAM, a relative intensity of each signal was constant irrespective of the positions in the sample to be measured.

Accordingly, it could be understood that a quantitative measurement could be done with good efficiency even by measuring one portion alone for a short period of time of 10 minutes or so, without measuring and integrated averaging the entire sample to be measured by subjecting to laser shots of 20,000 times or more for over 1 hour.

### Example 2

### <RNaseB glycopeptide, not labeled (derivatized)>

### <Example in which signal pattern of spectrum becomes the same by adding peptide even when glycan structures are heterogeneous>

First, 20 µg of RNaseB (available from Sigma-Aldrich Co.) was added to RapiGest SF (available from Waters Corp.) so that the final concentration became 0.1%, and the mixture was heated at 100°C for 5 minutes. After cooling, the mixture was dissolved in an aqueous solution containing 10 mmol/L ammonium bicarbonate and 10 mmol/L dithiothreitol, and incubated at 55°C for 45 minutes.

After the reaction, 5 µL of 135 mmol/L iodoacetamide was added to the mixture, and the resulting mixture was allowed to stand at a dark place for 45 minutes. Next, 1 µg of Lys-C was added to the mixture, and the resulting mixture was reacted at 37°C overnight.

The solution after the reaction was concentrated and purified by using cellulose, and 1 µL of the obtained solution was dropped onto a plate (sample-supporting member) for mass spectrometry, and the plate was allowed to stand at room temperature (23°C) under atmospheric pressure to dry it.

With regard to the sample to be measured, crystallization was carried out in the same manner as in Example 1, and a mass spectrum in a positive ion mode was obtained by the MALDI mass spectrometry method.

In Fig. 4, mass spectra measured at the different three points of the sample to be measured, distributions of the points at which signals were markedly detected of the glycopeptide (Man5 to 9-SRNSTK) of the molecules to be measured and a confocal laser microphotograph of the sample to be measured were shown.

"SRNSTK" represents a peptide shown by one-letter amino acid sequence.

### <Results of Example 2>

When the mass spectra of Figs. 4(a) to (c) of Example 2 are compared to each other, it can be understood that the signal strength ratio of the three spectra are constant irrespective of the measured places. Further, the signal distributions of Man5 to 7-SRNSTK were matched.

This shows that the respective signal distributions at the points each of which detects a signal are common if the peptide portions of the glycopeptides are common and even though the chemical structures of the glycan portions are different from each other.

Accordingly, it could be understood that a quantitative measurement could be done with good efficiency even by measuring one portion alone where a signal is detected for a short period of time of 10 minutes or so, without searching a point where a stronger signal is detected over time, without measuring and integrated averaging the entire sample to be measured by subjecting to laser shots of 20,000 times or more for over 1 hour.

### Example 3

### <IRNKS/GlcNAc-IRNKS/NA2-IRNKS, not labeled (derivatized)>

First, onto a plate (sample-supporting member) for mass spectrometry was dropped 1 µL of an aqueous solution which had been prepared by dissolving 1 pmol/µL of a peptide IRNKS, 500 fmol/µL of a glycopeptide GlcNAc-IRNKS, and 500 fmol/µL of NA2-IRNKS (chemical structures are shown in Fig. 9) which are molecules to be measured in water, and the plate was allowed to stand at room temperature (23°C) under atmospheric pressure to dry it. "IRNKS" represents a peptide shown by one-letter amino acid sequence.

With regard to the sample to be measured, crystallization of the matrix was carried out in the same manner as in Example 1, and a mass spectrum in a positive ion mode was obtained by the MALDI mass spectrometry method.

In Fig. 5, the respective positions at which signals of the peptide and glycopeptide ions which are the molecules to be measured in the sample to be measured were markedly detected and a confocal laser microphotograph of the sample to be measured were shown.

### <Results of Example 3>

When Fig. 5 of Example 3 is compared, signal distributions of the three molecules to be measured were matched.

This shows that the respective signal distributions are common if the peptide portions of the glycopeptides are common, irrespective of the presence or absence of the glycan portions and even though the chemical structures of the glycan portions are different from each other.

Accordingly, it could be understood that a quantitative measurement could be done with good efficiency even by measuring one portion alone for a short period of time of 10 minutes or so, without measuring and integrated averaging the entire sample to be measured by subjecting to laser shots of 20,000 times or more for over 1 hour.

### Example 4

### <IRNKS/GlcNAc-IRNKS/NA2-IRNKS, PDAM labeled (derivatized)>

### <When peptides are the same, and further being pyrene-labeled, signal pattern becomes more homogeneous irrespective of measured points>

First, onto a plate (sample-supporting member) for mass spectrometry was dropped 1 µL of an aqueous solution which had been prepared by dissolving 1 pmol/µL of peptide IRNKS, and each 500 fmol/µL of glycopeptide GlcNAc-IRNKS and NA2-IRNKS (chemical structures are shown in Fig. 9) which are molecules to be measured in water, and the plate was allowed to stand at room temperature (23°C) under atmospheric pressure to dry it.

"GlcNAc" represents N-acetylglucosamine.

Next, 0.25 µL of a solution which had been prepared by dissolving a labeling reagent PDAM (1-pyrenyldiazo-methan; available from Molecular Probes Inc.) which is a derivatizing agent in DMSO (dimethyl sulfoxide; available from SIGMA Co.), and making 10 nmol/µL was dropped onto the above, and the plate was allowed to stand on a heat block at 70°C under atmospheric pressure to dry it.

To remove excess derivatizing agent, the plate was dipped in xylene (available from SIGMA Co.), whereby excess PDAM was removed and the plate was sufficiently dried.

With regard to the sample to be measured, crystallization was carried out in the same manner as in Example 1, and a mass spectrum in a positive ion mode was obtained by the MALDI mass spectrometry method.

In Fig. 6, the respective positions at which signals of the peptide and glycopeptide ions which are the molecules to be measured in the sample to be measured were markedly detected and a confocal laser microphotograph of the sample to be measured were shown.

### <Results of Example 4>

When Fig. 6 of Example 4 is compared, distributions at the three points which detect signals of the molecules to be measured were matched, and were more homogeneous than the distribution state of Example 3.

This shows that the respective signal distributions at the points each of which detects a signal are common if the peptide portions of the glycopeptides are common and PDAM derivatization is performed, irrespective of the presence or absence of the glycan portions and even though the chemical structures of the glycan portions are different from each other.

Accordingly, it could be understood that a quantitative measurement could be done with good efficiency even by measuring one portion alone for a short period of time of 10 minutes or so, without measuring and integrated averaging the entire sample to be measured by subjecting to laser shots of 20,000 times or more for over 1 hour.

### Example 5

### <IgG1+IgG2 mixture, not labeled (derivatized)>

### <It is possible to examine a glycoform of IgG1 or IgG2>

First, 20 µg of immunoglobulin G (IgG, mainly subclass IgG1 and IgG2 are contained) (available from Wako Pure Chemical Industries, Ltd.) was added to RapiGest SF (available from Waters Corp.) so that the final concentration became 0.1%, and the mixture was heated at 100°C for 5 minutes.

After cooling, the mixture was dissolved in an aqueous solution containing 10 mmol/L ammonium bicarbonate and 10 mmol/L dithiothreitol, and incubated at 55°C for 45 minutes.

After the reaction, 5 µL of 135 mmol/L iodoacetamide was added to the mixture, and the resulting mixture was allowed to stand at a dark place for 45 minutes. To the solution after the reaction was added 1 µg of trypsin and the resulting mixture was reacted at 37°C overnight.

"IgG1" and "IgG2" are each subclass of immunoglobulin G, and show glycopeptides of the molecules to be measured obtained from an IgG reagent by the above-mentioned operation.

After the reaction, the solution was concentrated and purified by using cellulose, 0.8% trifluoroacetic acid was added to the resultant and the mixture was heated to 80°C for 45 minutes to evaporate the solvent.

The obtained sample was dissolved in water, 1 µL of the solution was dropped onto a plate (sample-supporting member) for mass spectrometry, and the plate was allowed to stand at room temperature (23°C) under atmospheric pressure to dry it.

With regard to the sample to be measured, crystallization was carried out in the same manner as in Example 1, and a mass spectrum in a negative ion mode was measured by the MALDI mass spectrometry method.

In Fig. 7(a), a confocal laser microphotograph of the sample to be measured was shown, in Fig. 7(b), a mass spectrum obtained in the sample to be measured was shown, and in Fig. 7(c), the respective positions at which signals (G0F, G1F and G2F of the respective IgG1 and IgG2) of the glycopeptide of the molecules to be obtained had been markedly detected were shown.

### Comparative Example 3

### <"IgG1+IgG2 mixture" same as in Example 5, not derivatized>

### <IgG1 and IgG2 different in peptide are uneven distribution and separated, and heterogeneous in MALDI image>

In the same manner as in Example 5 except for using the molecules to be measured in which the peptide portions are different from each other and the glycan portions are common, the same experiment as in Example 5 was carried out.

### <Results of Example 5 and Comparative Example 3>

In the mass spectrum shown in Fig. 7(b) of Example 5, six kinds of the glycopeptides (G0F, G1F and G2F of the respective IgG1 and IgG2) have been detected. The glycopeptides G0F, G1F and G2F of IgG2 among these are different in the chemical structure of the glycans but the peptide portions are common with EEQFNSTFR. Also, the glycopeptide G0F, G1F and G2F of IgG1 are different in the chemical structure of the glycans but the peptide portions are common with EEQYNSTYR.

When the signal distributions of these six kinds of glycopeptides are compared with each other, the distributions of the points which detect signals of the three molecules to be measured in which the peptide portions thereof are common are matched. That is, even if the glycans of G0F, G1F and G2F are different from each other, the peptide portions of the glycopeptide are the same, the signals thereof could be detected from the same position with good reproducibility.

This shows that the respective signal distributions at the points each of which detects a signal are common if the peptide portions of the glycopeptides are common and even though the chemical structures of the glycan portions are different from each other.

Accordingly, it could be understood that a quantitative measurement could be done with good efficiency even by measuring one portion alone for a short period of time of 10 minutes or so, without measuring and integrated averaging the entire sample to be measured by subjecting to laser shots of 20,000 times or more for over 1 hour.

On the other hand, in Comparative Example 3, when the signal distributions of the molecules to be measured in which the peptide portions are different from each other but the glycan portions are common are compared to each other, it could be understood that they were quite different from each other.

Accordingly, even when the glycans are the same, if the peptides are different from each other, the detected positions of the signals are also different from each other. Thus, it could be understood that the signal strengths of the glycopeptides in which the peptide portions are different from each other cannot be compared to each other by measuring one portion alone of the samples to be measured.

### Example 6

### <IgG1+IgG2 mixture, derivatized>

### <Sensitivity improved, spectrum patterns are the same, MALDI images are similar even when peptides are different, it is possible to compare IgG1 and IgG2 having different peptides>

First, 20 µg of immunoglobulin G (IgG) (available from Wako Pure Chemical Industries, Ltd.) was added to RapiGest SF (available from Waters Corp.) so that the final concentration thereof became 0.1%, and the mixture was heated at 100°C for 5 minutes. After cooling, the mixture was dissolved in an aqueous solution containing 10 mmol/L ammonium bicarbonate and 10 mmol/L dithiothreitol, and incubated at 55°C for 45 minutes.

After the reaction, 5 µL of 135 mmol/L iodoacetamide was added to the mixture, and the resulting mixture was allowed to stand at a dark place for 45 minutes. Next, 2 µg of chymotrypsin was added to the mixture and reacted at 25°C overnight.

After the reaction, the solution was concentrated and purified by using cellulose, 0.8% trifluoroacetic acid was added to the solution and the mixture was heated to 80°C for 45 minutes to evaporate the solvent.

The obtained sample was dissolved in water, 1 µL of the solution was dropped onto a plate (sample-supporting member) for mass spectrometry, and the plate was allowed to stand at room temperature (23°C) under atmospheric pressure to dry it.

Next, 0.25 µL of a solution which had been prepared by dissolving a labeling reagent PDAM (1-pyrenyldiazo-methan; available from Molecular Probes Inc.) which is a derivatizing agent in DMSO (dimethyl sulfoxide; available from SIGMA Co.), and making 10 nmol/µL was dropped onto the above, and the plate was allowed to stand on a heat block at 70°C under atmospheric pressure to dry it.

To remove excess derivatizing agent, the plate was dipped in xylene (available from SIGMA Co.), whereby excess PDAM was removed and the plate was sufficiently dried.

With regard to the sample to be measured, crystallization of the matrix was carried out in the same manner as in Example 1, and a mass spectrum in a negative ion mode was obtained by the MALDI mass spectrometry method.

In Fig. 8, a confocal laser microphotograph of the sample to be measured, and the respective positions from which signals (IgG1 (G1F), IgG2 (G1F)) of the glycopeptides of the molecules to be measured had been markedly detected were shown.

When the signal distributions of the glycopeptides of IgG1 and IgG2 of Example 6 were compared to each other, in either of the glycopeptides, strong signals were detected from the same portions of the sample to be measured.

The above shows that even when the peptide portions of the glycopeptides are different from each other, by derivatizing them using PDAM, not only detection sensitivities thereof are increased, but also different glycopeptides can be simultaneously measured from even one portion of the sample to be measured.

Accordingly, it could be understood that a quantitative measurement could be done with good efficiency even by measuring one portion alone for a short period of time, without measuring and integrated averaging the entire sample to be measured by subjecting to laser shots of 20,000 times or more for over 1 hour.

### Example 7

### <Mixture of Two kinds of glycopeptides, derivatized>

### <Sensitivity improved, spectrum patterns are the same, MALDI images are similar even when peptides are different, it is possible to compare NA2-LNDTR and NA2-AQNNGSN having different peptides>

First, 2 µg of α2-HS-glycoprotein (available from Sigma Co.) was dissolved in 50 mmol/L ammonium bicarbonate, thermolysin was added to the solution so that the final concentration became 200 U/mL, and the resulting mixture was incubated at 56°C overnight. After the reaction, 0.8% trifluoroacetic acid was added to the mixture and the resulting mixture was heated at 80°C for 45 minutes to evaporate the solvent. Next, water was added to the sample to dissolve again, and the sample was purified by using carbon graphite cartridge (available from GL Science). Further, the sample was concentrated and purified by using cellulose, then, the sample was dried by evaporating the solvent.

The obtained sample was dissolved in water, 1 µL (1 pmol) of the solution was dropped onto a plate (sample-supporting member) for mass spectrometry, and the plate was allowed to stand at room temperature (23°C) under atmospheric pressure to dry it.

Next, in the same manner as in Example 6, derivatiza-tion of the glycopeptide by PDAM was carried out.

With regard to the sample to be measured, crystallization of the matrix was carried out in the same manner as in Example 1, and a mass spectrum in a negative ion mode was obtained by the MALDI mass spectrometry method.

When signal distributions of two kinds of glycopeptides NA2-LNDTR and NA2-AQNNGSN (chemical structures are shown in Fig. 9) having different peptides obtained in Example 7 were compared to each other, in either of the glycopeptides, strong signals were equally detected from the sample to be measured, and the distributions of the strong signals were the same. Further, in the sample to be measured, when the mass spectra obtained from the different measurement points were compared to each other, the relative intensities of the signals of two molecules to be measured were constant irrespective of the places to which a laser was irradiated, there was no fluctuation in the same sample to be measured, and accordingly, the respective spectra at each point were matched with the spectrum obtained by automatically measuring and integrated averaging the entire sample to be measured.

### Example 8

### <Mixture of various kinds of glycopeptides, derivatized>

### <Sensitivity improved, spectrum patterns are the same, MALDI images are similar even when peptides are different, it is possible to compare site-specific glycan structures>

After 10 µg of α1-acid-glycoprotein (available from Sigma Co.) was modified by RapiGest in the same manner as in Example 6, reduction alkylation was carried out, and the reaction mixture was dissolved in 50 mmol/L ammonium bicarbonate, Glu-C was added thereto so that it became 500 ng (1/20 of the protein amount), and the resulting mixture was incubated at 37°C overnight.

After the reaction, 0.8% trifluoroacetic acid was added to the mixture and the resulting mixture was heated at 80°C for 45 minutes to evaporate. Next, water was added to the sample to dissolve it again, and the sample was concentrated and purified by using cellulose, then, the sample was dried by evaporating the solvent.

The obtained sample was dissolved in water, 1 µL (250 ng) thereof was dropped onto a plate (sample-supporting member) for mass spectrometry, and the plate was allowed to stand at room temperature (23°C) under atmospheric pressure to dry it.

Next, in the same manner as in Example 6, derivatization of the glycopeptide by PDAM was carried out.

With regard to the sample to be measured, crystallization of the matrix was carried out in the same manner as in Example 1, mass spectra in positive ion and negative ion modes were obtained by the MALDI mass spectrometry method.

In Fig. 10, a negative ion spectrum obtained by irradiating a laser to one portion in the matrix crystal of the Glu-C digested-glycopeptide mixture is shown.

Glycopeptides containing four sites of the glycan-bonded asparagines of N38, N54, N75 and N85 were detected. It could be understood that glycans NA2 and NA3 were bonded to N38, glycans NA2, NA3 and NA4 were bonded to N54, glycans NA3 and NA4 were bonded to N75, and glycans NA2, NA3 and NA4 were bonded to N85.

Further, in Fig. 11, the entire matrix was automatically measured, and of these, a distribution of the signals of the glycopeptides was shown, and they were the same distributions. That is, the distributions are the same both in the glycopeptides in which the peptides are the same but the glycans are different from each other and in the glycopeptides in which the peptides are different from each other, which show that the spectra at the respective measurement points are the same.

The same results can be obtained by a positive ion measurement.

### Comparative Example 4

### <Mixture of various kinds of glycopeptides, not derivatized>

In the same manner as in Example 8, after α1-acid-glycoprotein was subjected to Glu-C digestion, MALDI mass spectrometry of the sample to be measured which had not been subjected to PDAM derivatization was carried out. As a result, the glycopeptides containing the respective four sites of the glycan-bonded asparagines detected in Example 8 could not be detected, and a quantitative MS spectrum could not be obtained.

This means that, in the glycopeptide mixture in which the glycan is bonded to the different peptides, not to one kind of the peptide, these glycopeptides having the respective peptides act with the matrix molecule with different interaction, or inhibit ionization with each other, which make the quantitative measurement difficult.

### Example 9

### <Mixture of various kinds of glycopeptides, derivatized>

The similar results could be obtained in the thermolysin-digested glycopeptides mixture obtained by subjecting α1-acid-glycoprotein to the same manner as in Example 6. That is, glycopeptides in which a plural kinds of glycans had been bonded to four kinds of the peptides of ITN containing N15, FTPNKTEDT containing N54, IYN containing N75, and VQRENGT containing N85 could be detected ("ITN", "FTPNKTEDT", "IYN" and "QRENGT" each represent a peptide shown by one-letter amino acid sequence.), and the respective signal distributions were the same.

As mentioned above, it is preferred in the point that the glycopeptide sample prepared from a glycoprotein is a mixture in which a glycan portion and/or a peptide portion is/are different from each other, and according to the present invention, by preparing a sample to be measured by crystallizing molecules to be measured labeled with a condensed polycyclic compound and a matrix, a point which detects a signal becomes common with respect to the multiple molecules to be measured in "a mixed crystal or a mixture of a sample and a matrix" which is a sample to be measured prepared on a sample-supporting member, so that if a laser is irradiated to the point which detects the signal, a quantitative mass spectrum for the multiple molecules to be measured can be obtained.

In particular, a sample to be measured derived from a living body sample contains many kinds of glycoproteins, or even when it is one kind of glycoprotein, glycans are bonded to two or more portions so that it is a mixture in which glycan portions and/or peptide portions are each different from each other, to which marked effects can be accomplished by the present invention.

Further, when thermolysin is used for the preparation of a glycopeptide, a relatively low molecular weight peptide is generated, so that it is preferred since occupancy of the common derivatized structure in the molecules to be measured labeled with the condensed polycyclic compound becomes large.

Or else, when digestion with Glu-C, Lys-C or Arg-C is performed, C-terminal amino acid becomes common, so that it is preferred since the effects by the common derivatized structure are further improved.

The above shows that even when the peptide portions of the glycopeptides are markedly different from each other, by subjecting to derivatization of these using PDAM, not only respective ionization efficiencies are equally increased and detection sensitivities are also increased, but also different glycopeptides can be simultaneously measured by one portion of the sample to be measured.

Accordingly, it could be understood that a quantitative measurement could be done with good efficiency even by measuring one portion alone which detects a signal firstly measured for a short period of time, without measuring and integrated averaging the entire sample to be measured by subjecting to laser shots of 20,000 times or more for over 1 hour, and without seeking the point which detects stronger signal of the respective molecules.

### INDUSTRIAL APPLICABILITY

The mass spectrometry method of the present invention can measure multiple molecules that are contained in a sample quantitatively within a short period of time with good efficiency. According to the above, this mass spectrometry method can obtain information concerning the chemical structures with high reliability, and this method is widely used not only in chemical structure analysis of molecules derived from a living body or molecules in a living body sample with a minute amount, but also in a field of elucidation of the function or elucidation of pathological conditions.

## Claims

1. A MALDI mass spectrometry method for a sample to be measured containing multiple different molecules, said sample to be measured being prepared from a sample containing multiple different molecules to be measured and a matrix, said method comprising the steps of
(i) providing the sample containing the multiple different molecules to be measured , said multiple different molecules being selected from the group consisting of a saccharide mixture, a glycopeptide mixture, a glycopeptide-peptide mixture, a glycoprotein mixture, and a glycoprotein-protein mixture, wherein either
a) the multiple different molecules to be measured are reacted with a common derivatizing agent, said derivatizing agent being a condensed polycyclic compound having a reactive functional group capable of bonding the condensed polycyclic portion and the molecules to be measured; or
b) the multiple different molecules to be measured have a common peptide or protein portion;
(ii) obtaining a quantitative mass spectrum of the multiple different molecules to be measured from an arbitrary point in the sample to be measured where a signal is detected without measuring and integrated averaging the entire sample to be measured.

2. The MALDI mass spectrometry method according to claim 1, wherein the multiple molecules to be measured are a glycopeptide mixture, peptide portions of the glycopeptide are the same with each other, or are a glycopeptide-peptide mixture, peptide portions of the glycopeptide are the same with each other, and the peptide portion of the glycopeptide and the peptide are the same with each other.

3. The MALDI mass spectrometry method according to claim 1, wherein the multiple molecules to be measured are a saccharide mixture, and a sample to be measured is prepared by reacting the sample with a condensed polycyclic compound, and crystallizing the obtained molecules to be measured labeled with the condensed polycyclic compound and a matrix.

4. The MALDI mass spectrometry method according to claim 1, wherein the multiple molecules to be measured are a glycopeptide-peptide mixture, the peptide portions of the glycopeptide are the same with each other, the peptide portion of the glycopeptide and the peptide are the same with each other, and a sample to be measured is prepared by reacting the sample with a condensed polycyclic compound and crystallizing the obtained molecules to be measured labeled with the condensed polycyclic compound and a matrix.

5. The MALDI mass spectrometry method according to claim 1, wherein the multiple molecules to be measured are a glycopeptide mixture, saccharides and/or peptides of the glycopeptides may be different from each other, and a sample to be measured is prepared by reacting the sample with a condensed polycyclic compound, and crystallizing the obtained molecules to be measured labeled with the condensed polycyclic compound and a matrix.

6. The MALDI mass spectrometry method according to any one of claim 1 or claim 3 to claim 5, wherein the condensed polycyclic compound is a pyrene ring compound.

7. The MALDI mass spectrometry method according to claim 6, wherein the pyrene ring compound is 1-pyrenyldiazomethane (PDAM).

8. The MALDI mass spectrometry method according to any one of claim 1 to claim 7, wherein the multiple molecules to be measured existing in a sample to be measured are each 10 pmol or less.

## Patentansprüche

1. MALDI-Massenspektrometrieverfahren für eine zu messende Probe, die mehrere unterschiedliche Moleküle enthält, wobei die zu messende Probe aus einer Probe, die mehrere unterschiedliche zu messende Moleküle enthält, und einer Matrix präpariert ist, wobei das Verfahren die Schritte umfasst:
(i) Bereitstellen der Probe, die die mehreren unterschiedlichen zu messenden Moleküle enthält, wobei die mehreren unterschiedlichen Moleküle aus der Gruppe ausgewählt sind, die aus einem Saccharidgemisch, einem Glycopeptidgemisch, einem Glycopeptid-Peptid-Gemisch, einem Glycoproteingemisch und einem Glycoprotein-Protein-Gemisch besteht, wobei entweder
a) die mehreren unterschiedlichen zu messenden Moleküle mit einem gemeinsamen Derivatisierungsmittel umgesetzt werden, wobei das Derivatisierungsmittel eine kondensierte polycyclische Verbindung ist, die eine reaktive funktionelle Gruppe aufweist, welche den kondensierten polycyclischen Teil und die zu messenden Moleküle binden kann; oder
b) die mehreren unterschiedlichen zu messenden Moleküle einen gemeinsamen Peptid- oder Proteinteil aufweisen;
(ii) Erhalten eines quantitativen Massenspektrums der mehreren unterschiedlichen zu messenden Moleküle ausgehend von einem beliebigen Punkt in der zu messenden Probe, wo ein Signal nachgewiesen wird, ohne zu messen, und integriert wird, wobei die gesamte zu messende Probe gemittelt wird.

2. MALDI-Massenspektrometrieverfahren gemäß Anspruch 1, wobei die mehreren zu messenden Moleküle ein Glycopeptidgemisch sind, die Peptidteile des Glycopeptids untereinander gleich sind, oder ein Glycopeptid-Peptid-Gemisch sind, die Peptidteile des Glycopeptids untereinander gleich sind und der Peptidteil des Glycopeptids und des Peptids untereinander gleich sind.

3. MALDI-Massenspektrometrieverfahren gemäß Anspruch 1, wobei die mehreren zu messenden Moleküle ein Saccharidgemisch sind und die zu messende Probe präpariert wird, indem man die Probe mit einer kondensierten polycyclischen Verbindung umsetzt und die erhaltenen zu messenden Moleküle, die mit der kondensierten polycyclischen Verbindung markiert sind, und eine Matrix kristallisiert.

4. MALDI-Massenspektrometrieverfahren gemäß Anspruch 1, wobei die mehreren zu messenden Moleküle ein Glycopeptid-Peptid-Gemisch sind, die Peptidteile des Glycopeptids untereinander gleich sind, der Peptidteil des Glycopeptids und des Peptids untereinander gleich sind und die zu messende Probe präpariert wird, indem man die Probe mit einer kondensierten polycyclischen Verbindung umsetzt und die erhaltenen zu messenden Moleküle, die mit der kondensierten polycyclischen Verbindung markiert sind, und eine Matrix kristallisiert.

5. MALDI-Massenspektrometrieverfahren gemäß Anspruch 1, wobei die mehreren zu messenden Moleküle ein Glycopeptidgemisch sind, die Saccharide und/oder Peptide der Glycopeptide voneinander verschieden sein können und die zu messende Probe präpariert wird, indem man die Probe mit einer kondensierten polycyclischen Verbindung umsetzt und die erhaltenen zu messenden Moleküle, die mit der kondensierten polycyclischen Verbindung markiert sind, und eine Matrix kristallisiert.

6. MALDI-Massenspektrometrieverfahren gemäß einem der Ansprüche 1 oder 3 bis 5, wobei die kondensierte polycyclische Verbindung eine Pyrenringverbindung ist.

7. MALDI-Massenspektrometrieverfahren gemäß Anspruch 6, wobei es sich bei der Pyrenringverbindung um 1-Pyrenyldiazomethan (PDAM) handelt.

8. MALDI-Massenspektrometrieverfahren gemäß einem der Ansprüche 1 bis 7, wobei die mehreren zu messenden Moleküle, die in einer zu messenden Probe vorliegen, jeweils 10 pmol oder weniger ausmachen.

## Revendications

1. Procédé de spectrométrie de masse MALDI (désorption-ionisation laser assistée par matrice) pour un échantillon à mesurer contenant de multiples molécules différentes, ledit échantillon à mesurer étant préparé à partir d'un échantillon contenant de multiples molécules différentes à mesurer et une matrice, ledit procédé comprenant les étapes consistant à :
(i) fournir l'échantillon contenant les multiples molécules différentes à mesurer, lesdites multiples molécules différentes étant sélectionnées dans le groupe composé d'un mélange de saccharides, d'un mélange de glycopeptides, d'un mélange de glycopeptides-peptides, d'un mélange de glycoprotéines et d'un mélange de glycoprotéines-protéines, où
a) soit les multiples molécules différentes à mesurer sont mises à réagir avec un agent commun de dérivation, ledit agent de dérivation étant un composé polycyclique condensé ayant un groupe fonctionnel réactif capable de lier la portion polycyclique condensée et les molécules à mesurer ;
b) soit les multiples molécules différentes à mesurer ont un peptide ou une portion de protéine commun(e) ;
(ii) obtenir un spectre de masse quantitatif des multiples molécules différentes à mesurer à partir d'un point arbitraire dans l'échantillon à mesurer où un signal est détecté sans mesure ni moyenne intégrée de l'ensemble de l'échantillon à mesurer.

2. Procédé de spectrométrie de masse MALDI selon la revendication 1, dans lequel les multiples molécules à mesurer sont un mélange de glycopeptides, les portions de peptides du glycopeptide sont les mêmes, ou sont un mélange de glycopeptides-peptides, les portions peptide du glycopeptide sont les mêmes, et la portion peptide du glycopeptide et du peptide sont les mêmes.

3. Procédé de spectrométrie de masse MALDI selon la revendication 1, dans lequel les multiples molécules à mesurer sont un mélange de saccharides, et un échantillon à mesurer est préparé en faisant réagir l'échantillon avec un composé polycyclique condensé, et en cristallisant les molécules à mesurer obtenues marquées avec un composé polycyclique condensé et une matrice.

4. Procédé de spectrométrie de masse MALDI selon la revendication 1, dans lequel les multiples molécules à mesurer sont un mélange de glycopeptides-peptides, les portions peptide du glycopeptide sont les mêmes, la portion peptide du glycopeptide et du peptide sont les mêmes, et un échantillon à mesurer est préparé en faisant réagir l'échantillon avec un composé polycyclique condensé et en cristallisant les molécules obtenues à mesurer marquées avec le composé polycyclique condensé et une matrice.

5. Procédé de spectrométrie de masse MALDI selon la revendication 1, dans lequel les multiples molécules à mesurer sont un mélange de glycopeptides, les saccharides et/ou les peptides des glycopeptides peuvent être différents les uns des autres, et un échantillon à mesurer est préparé en faisant réagir l'échantillon avec un composé polycyclique condensé, et en cristallisant les molécules obtenues à mesurer marquées avec le composé polycyclique condensé et une matrice.

6. Procédé de spectrométrie de masse MALDI selon l'une quelconque des revendications 1 ou de la revendication 3 à la revendication 5, dans lequel le composé polycyclique condensé est un composé cyclique pyrène.

7. Procédé de spectrométrie de masse MALDI selon la revendication 6, dans lequel le composé cyclique pyrène est le 1-pyrényldiazométhane (PDAM).

8. Procédé de spectrométrie de masse MALDI selon l'une quelconque des revendications 1 à 7, dans lequel les multiples molécules à mesurer existant dans un échantillon à mesurer sont chacune de 10 pmol ou moins.
